# EUROPÄISCHE PATENTSCHRIFT

(11) **EP 1 186 589 B1**
(45) Veröffentlichungstag und Bekanntmachung des Hinweises auf die Patenterteilung: **05.11.2003**
(21) Anmeldenummer: 01120387.4
(22) Anmeldetag: 25.08.2001
(51) Int. Cl.: C07C 41/56, C07C 41/58, C07C 43/303

(54) **Kontinuierliches Verfahren zur Herstellung von Acetalen**
Continuous process for the production of acetals
Procédé en continue pour la production d'acétals

(30) Priorität: 07.09.2000 DE 10044352
(43) Veröffentlichungstag der Anmeldung: 13.03.2002
(73) Patentinhaber: BASF AKTIENGESELLSCHAFT, 67056 Ludwigshafen (DE)
(72) Erfinder: Therre, Jörg, Dr., 67551 Worms (DE); Kaibel, Gerd, Dr., 68623 Lampertheim (DE); Aquila, Werner, Dr., 68309 Mannheim (DE); Wegner, Günter, Dr., 67354 Römerberg (DE); Fuchs, Hartwig, 67063 Ludwigshafen (DE)

(56) Entgegenhaltungen:
- DE-A- 2 625 074
- US-A- 2 501 144

## Beschreibung

Die vorliegende Erfindung betrifft ein kontinuierliches Verfahren zur Herstellung von ungesättigten Acetalen durch Umsetzen von olefinisch ungesättigten aliphatischen Verbindungen mit Allylalkoholen in einer Reaktionskolonne, wobei die Reaktanten in der Reaktionskolonne nur teilweise umgesetzt werden, das erhaltene Acetal in mindestens zwei aufeinanderfolgenden Verdampferstufen aufkonzentriert wird und die zurückgewonnenen Reaktanten wieder in die Reaktionskolonne zurückführt werden.

Die Herstellung von ungesättigten Acetalen durch Umsetzen von olefinisch ungesättigten aliphatischen Verbindungen mit Allylalkoholen in einer Reaktionskolonne in Gegenwart einer destillierbaren Säure ist an sich aus DE 26 25 074 bekannt. Dort wird beschrieben, daß eine Mischung aus mindestens 2 Mol des Alkohols und einem Mol des Aldehyds in die Reaktionskolonne eingebracht und das bei der Reaktion entstehende Wasser über Kopf abdestilliert und mittels eines Phasenscheiders abgezogen werden soll. Aus dem Verdampfer der Kolonne soll dann das Acetal als Rohprodukt entnommen werden. Die Reaktionskolonne soll so betrieben werden, daß im Sumpfablauf der Kolonne kein Aldehyd mehr enthalten ist, der Aldehyd also vollständig in der Reaktionskolonne umgesetzt wird. In den Beispielen werden Umsätze für den Aldehyd von mehr als 94,5 genannt.

Das beschriebene Verfahren stellt einen großen Fortschritt für die Herstellung von Acetalen dar, weist aber leider einige Nachteile auf. Da nach dem bekannten Stand der Technik hohe Umsätze von über 90 % angestrebt werden, ist die Produktionsanlage nur schwer zu regeln. Geringe Schwankungen in der Menge der Zuflüsse oder in den Reinheiten der Einsatzstoffe führen dazu, daß der gewünschte Umsatz in der Reaktionskolonne nicht eingehalten werden kann. Besonders macht sich dieser Umstand beim Einsatz von verunreinigten Einsatzstoffen bemerkbar. Solche Verunreinigungen sind z.B. Folgeprodukte des Aldehyds und des Alkohols wie z.B. Formiate des Alkohols, aus dem Alkohol gebildete Ether oder über C-C Bindungen verknüpfte Kondensationsprodukte aus Alkohol und Aldehyd. Solche Nebenprodukte entstehen bekanntlich insbesondere dann in größerer Menge, wenn das Acetal einer Spaltung- und Umlagerungsreaktion nach Claisen und Cope unterzogen wird, wie dies z.B. bei der Herstellung von Citral der Fall ist.

Besonders schwierig gestaltet sich die Zugabe der richtige Menge an Säure. Hier kann schon eine geringe Unterdosierung der Säuremenge zum Zusammenbrechen des Umsatzes in der Reaktionskolonne führen. Wird aber mehr als die richtige Menge an Säure zugegeben, führt dies zu einem starken Ansteigen der Menge an schwersiedenden Nebenkomponenten und Ethern. Erschwert wird die Regelung der Säuremenge dadurch, daß sich die Säure in der Reaktionskolonne ansammelt, und dadurch eine Über- oder Unterdosierung erst mit großer zeitlicher Verzögerung bemerkt wird.

Die oben genannten Nachteile haben die wirtschaftliche Herstellung der Acetale in einer Reaktionskolonne im technischen Maßstab bisher verhindert.

Aufgabe der Erfindung war es, den genannten Nachteile abzuhelfen und ein gut zu regelndes, stabil zu betreibendes Verfahren zur Herstellung von ungesättigten Acetalen zu schaffen.

Die Aufgabe wurde erfindungsgemäß gelöst, durch ein Verfahren zur Herstellung von ungesättigten Acetalen der allgemeinen Formel I 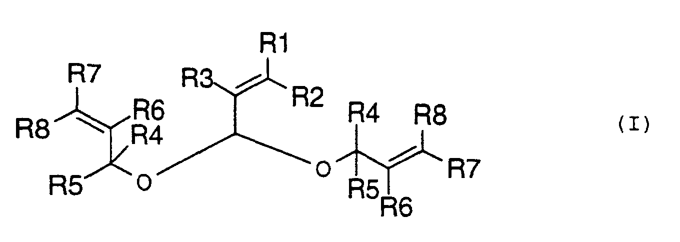 in der
R1 bis R7 unabhängig voneinander Wasserstoff, einen geradkettigen oder verzweigten, gegebenenfalls substituierten C₁-C₆-Alkylrest bedeuten und
R8 Wasserstoff, einen gesättigten oder einen ein oder mehrfach ungesättigten, geradkettigen oder verzweigten, gegebenenfalls substituierten C₁-C₁₂-Alkylrest oder einen gegebenenfalls substituierten 3 bis 12-gliedrigen gesättigten oder einen ein oder mehrfach ungesättigten Carbocyclus bedeutet,
durch Umsetzen von einem Mol eines Aldehyds der Formel II 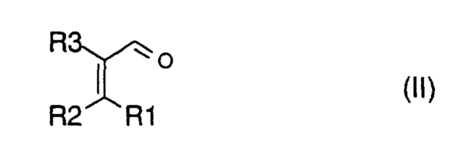 in der R1 bis R3 die oben angegebene Bedeutung haben mit mindestens 1 mol eines Alkohols der Formel III 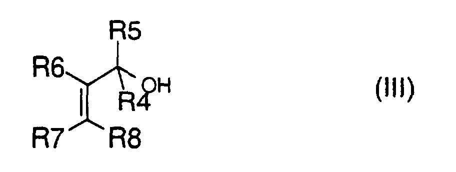 in der R5 bis R8 die oben angegebene Bedeutung haben, in Gegenwart katalytischer Mengen an Säure und unter Abtrennung des bei der Reaktion gebildeten Wassers, dadurch gekennzeichnet daß man die Reaktanten in einer Reaktionskolonne nur teilweise umsetzt, das erhaltene Acetal in mindestens zwei aufeinanderfolgenden Verdampferstufen aufkonzentriert und die zurückgewonnenen Reaktanten wieder in die Reaktionskolonne zurückführt.

Unter einem geradkettigen oder verzweigten C₁-C₆-Alkylrest versteht man beispielsweise einen Methyl-, Ethyl-, Propyl-, Isopropyl-, Butyl-, tert.-Butyl-, Pentyl- oder Hexylrest, vorzugsweise einen Methyl- oder Isopropylrest.

Unter einem gesättigten oder ein oder mehrfach ungesättigten geradkettigen oder verzweigten C₁-C₁₂-Alkylrest versteht man beispielsweise einen Methyl-, Ethyl-, Propyl-, Isopropyl-, Butyl-, tert.-Butyl-, Pentyl-, Hexyl-, Allyl-, Ethylen-, Propylen-, Butylen-, Isobutylen, Pentenyl-, Hexenyl-, Heptenyl-, Octenyl-, Decenyl-, Acetylen-, Propinyl-, Butinyl-, Butadienyl-, Isoprenyl- oder den Hexadienylrest, vorzugsweise den Methyl- oder Pentenylrest.

Unter einem 3 bis 12-gliedrigen gesättigten oder ein oder mehrfach ungesättigten Carbocyclus versteht man beispielsweise einen Cyclopropyl-, Cyclobutanyl-, Cyclopentyl-, Cyclohexyl-, Cycloheptyl-, Cycloocten-, Cyclohexen-, Cyclopenten-, Cyclooctadien, Cyclooctatetraen- oder einen Cyclododecatrienrest.

Unter den Substituenten eines carbocyclischen Ringsystems oder eines Alkylrestes versteht man beispielsweise Halogen, Nitro, Cyano, Hydroxy, C₁-C₆-Alkyl, C₁-C₆-Alkoxy, C₁-C₆-Alkoxycarbonyl oder Amino.

Alkoxygruppen bedeuten in Kombination mit einer Alkylgruppe gemäß der obigen Definition, mit einem Sauerstoffatom, z.B. Methoxy, Ethoxy, Propoxy, Isopropoxy, Butoxy oder Pentoxy, vorzugsweise Methoxy.

Als Aldehyde der Formel II können beispielsweise Acrolein, 2-Buten-1-al, 2-Methyl-2-buten-1-al, 3-Methyl-2-buten-1-al, 2-Methyl-4-methoxy-2-buten-1-al, 2-Methyl-4-methoxy-2-buten-1-al, 3-Isopropyl-2-buten-1-al eingesetzt werden. Von besonderer Bedeutung für weitere Synthesen sind Acetale die sich vom 3-Methyl-2-buten-1-al ableiten.

Erfindungsgemäß geeignete Alkohole der Formel III sind beispielsweise 2-Propen-1-ol, 2-Buten-1-ol, 2-Methyl-3-buten-2-ol, 3-Methyl-2-buten-1-ol, Geraniol, 2-Methyl-2-propen-1-ol, vorzugsweise 3-Methyl-2-buten-1-ol (Prenol).

Die nach dem Verfahren der Erfindung erzeugten Acetalen sind wertvolle Ausgangsstoffe für Kunststoffe, Wirkstoffe, Riechstoffe und Vitamine. So ist das 1,1-Bis-(3-methyl-2-buten-1-yloxy)-3-methyl-2-buten (3-Methyl-2-butenal-diprenylacetal) eine wichtige Ausgangsverbindung des Riechstoffes Citral.

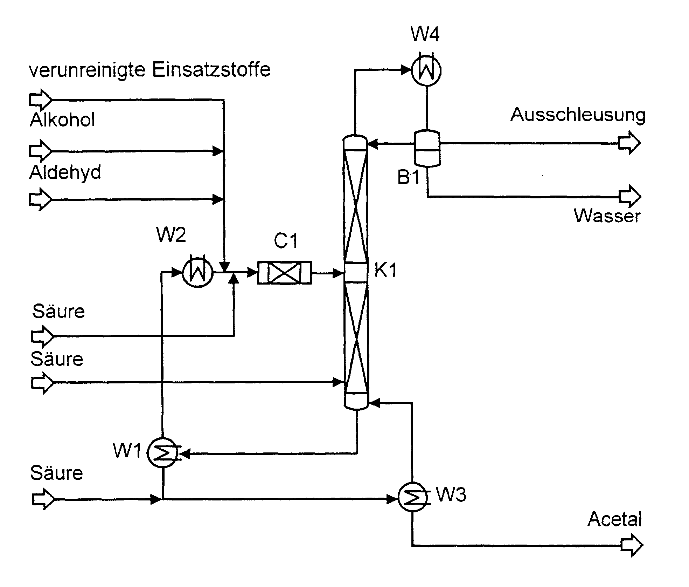

Das Verfahren ist in der Abbildung schematisch dargestellt und wird im folgenden beschrieben.

Die Apparatur besteht aus einer Destillationskolonne K1, die als Reaktionskolonne benutzt wird. Die am Kopf der Kolonne aufsteigenden Dämpfe werden in dem Kondensator W4 kondensiert und in ein Phasentrenngefäß B1 geleitet, in dem sich das Wasser als untere Phase abscheidet. Die obere Phase besteht im Wesentlichen aus organischen Verbindungen (Aldehyd, Alkohol und leichtsiedenden Nebenverbindungen, z.B. den Formiaten des eingesetzten Alkohols). Der größte Teil der organischen Phase wird als Rückfluß auf den Kopf der Kolonne K1 zurückgeführt, ein kleinerer Teil zur Entfernung der Nebenkomponenten ausgeschleust.

Die Menge des Rückflusses beträgt pro 1000 kg frisch zugegebenem Aldehyd 200 kg bis 50000 kg, insbesondere 1000 kg bis 20000 kg. Diese geringe Menge an Rückfluß und damit der niedrige Energiebedarf stellt einen besonderen Vorteil des erfindungsgemäßen Verfahrens dar.

Die Menge an Ausschleusung beträgt abhängig von der Reinheit der verwendeten Einsatzstoffe pro 1000 kg frisch zugegebenem Aldehyd zwischen 1 kg und 400 kg, insbesondere zwischen 5 kg und 200 kg.

Der Sumpfablauf der Kolonne gelangt in den Verdampfer W1, der die erste Stufe der zweistufigen Aufkonzentrierung des Acetals darstellt.

Die im Verdampfer W1 gewonnen Dämpfe bestehen aus 10 Gew% bis 80 Gew% Alkohol, bis zu 10 Gew% des Acetals und 10 Gew% bis 40 Gew% des Aldehyds. Der Umsatz an Aldehyd in der Reaktionskolonne liegt also unter 90 %. Es ist möglich auf die Kondensation der Dämpfe zu verzichten und die Dämpfe gasförmig in die Kolonne K1 zurückzueilen. Vorzugsweise werden die Dämpfe aber in dem Kondensator W2 kondensiert.

Die Menge der in der Verdampferstufe W1 erzeugten Dämpfe, das heißt die Menge der in der ersten Verdampferstufe zurückgeführten Reaktanten, beträgt zwischen dem doppelten und dem dreißigfachen, insbesondere zwischen dem dreifachen und dem zwanzigfachen der Menge des frisch zugegebenen Aldehyds. Eine zu geringe Menge der in der ersten Verdampferstufe zurückgeführten Reaktanten führt zu einem Selektivitätsverlust. Eine zu hohe Menge der in der ersten Verdampferstufe zurückgeführten Reaktanten ist zwar für die Selektivität der Acetalsynthese günstig, verbraucht aber unnötig hohe Energiemengen. Ein großer Vorteil des erfindungsgemäßen Verfahrens ist die leichte Regelbarkeit und der stabile Betrieb, da die Menge der in der ersten Verdampferstufe zurück zu führenden Reaktanten leicht durch Beobachten der Temperaturen in der Kolonne K1 und den Verdampfern W1 und W3 bestimmt werden kann.

Gegebenenfalls kann ein Reaktor C1 verwendet werden, in den das im Kondensator W2 gewonnene Kondensat und die Einsatzstoffe Alkohol und Aldehyd zugegeben werden. Der Reaktor C1 dient zur Einstellung des thermodynamischen Gleichgewichtes zwischen Alkohol und Aldehyd einerseits und Wasser und Acetal andererseits. Für den Reaktor C1 können rückvermischte Reaktoren (wie z.B. Rührkessel) aber vorteilhaft nicht rückvermischte Reaktoren mit Rohrreaktorcharakteristik (wie z.B. mit Füllkörperkolonnen, kaskadierte Behälter) verwendet werden. Die Verweilzeit des Reaktionsgemisches im Reaktor C1 soll zwischen 0,1 sec und 10 Stunden betragen. Da sich das thermodynamische Gleichgewicht oft sehr schnell einstellt, reicht unter Umständen eine sehr kurze Verweilzeit aus. Dann kann gegebenenfalls auf einen speziellen Apparat verzichtet werden, und die ohnehin vorhandene Rohrleitung zwischen der Mischstelle von Alkohol, Aldehyd und dem im Kondensator W2 gewonnenen Kondensat als Reaktor C1 dienen. Gegebenenfalls können zum Vermischen der Einsatzstoffe mit dem dem im Kondensator W2 gewonnenen Kondensat die üblichen Mischorgane wie z.B. statische Mischer oder Rührkessel verwendet werden.

Die Säure kann in den Reaktor C1 oder den Verdampfer W1 zugegeben werden. Bevorzugt wird die Säure aber in die Reaktionskolonne K1 zugegeben und dort wieder mit Vorteil im unteren Teil der Kolonne oder in den Verdampfer W3 zugegeben, wenn es sich um eine flüchtige und insbesondere um Salpetersäure handelt. Es ist auch möglich die Säure an verschiedenen Stellen zuzugeben, z.B. an zwei oder mehr Stellen in der Kolonne oder teilweise in die Kolonne und teilweise in den Reaktor C1. Die aus dem Reaktor austretende Flüssigkeit soll bevorzugt im oberen Teil der Kolonne zugegeben werden. Es ist auch möglich die Flüssigkeit, gegebenenfalls vermischt mit dem Rückfluß, direkt als Rücklauf auf den Kopf der Kolonne zuzugeben.

Die Zugabestelle von frischem Aldehyd und frischem Alkohol ist nicht kritisch. Die beiden Einsatzstoffe können auch getrennt an verschiedenen Stellen der Kolonne K1 und/oder dem Reaktor C1 zugegeben werden. Bevorzugt werden die Einsatzstoffe mit dem Ablauf aus dem Kondensator W2 vereinigt. Die zugegebene Menge an frisch zugegebenem Alkohol wird so geregelt, dass das Verhältnis aus Alkohol zu aldehyd zwischen 1 und 3, insbesondere zwischen 1,5 und 2,5 beträgt. Ein besonderer Vorteil des erfindungsgemäßen Verfahrens besteht darin, daß auch die oben beschriebenen verunreinigten Einsatzstoffe problemlos verwendet werden können.

Die aus dem Verdampfer W1 austretende Flüssigkeit enthält das Acetal in der Regel in einer Konzentration zwischen 10 Gew.-% und 70 Gew.-%. Der Rest besteht im Wesentlichen aus dem Aldehyd und dem Alkohol. Diese Flüssigkeit wird in den Verdampfer W3 gegeben und dort das Acetal in einer Konzentration zwischen 30 Gew.-% und 99,9 Gew.-% als Flüssigkeit gewonnen, insbesondere von 50 Gew.-% bis 95 Gew.-%. Die aus dem Verdampfer W3 aufsteigenden Dämpfe werden bevorzugt wieder in den Sumpf der Kolonne K1 zurückgeführt und somit zur Beheizung der Kolonne verwendet.

Als Einbauten für die Kolonne eigenen sich Blechpackungen (z.B. Sulzer 250Y), Gewebepackungen (Sulzer BX oder CY) oder Böden (z.B. Glockenböden, Ventilböden, Tunnelböden. Die Zahl der theoretischen Trennstufen in der Reaktionskolonne soll zwischen 3 bis 80 und insbesondere zwischen 5 und 40 betragen.

Der Kopfdruck in der Kolonne K1 liegt zwischen 2 mbar und 400 mbar, insbesondere soll der Druck aber so gewählt werden, daß sich am Kopf eine Kondensationstemperatur über 0°C, bevorzugt über 20°C und ganz besonders über 35°C ergibt. Der Druck im Verdampfer W3 ergibt sich in der bevorzugten Ausgestaltung des Verfahrens aus dem Differenzdruck der Kolonne K1. Der Druck im Verdampfer W1 liegt zwischen 2 mbar und 400 mbar

Die Temperatur im Reaktor C1 soll zwischen -20°C und 100°C, insbesondere zwischen 0°C und 60°C betragen.

Als Säuren können unter den Reaktionsbedingungen nicht flüchtige Säuren verwendet werden. Diese Säuren werden bevorzugt im oberen Teil der Reaktionskolonne zugegeben. Im unteren Teil der Kolonne oder vor/in den Verdampfern W1 oder W3 kann dann die zugegebene Säure wieder durch Neutralisation entfernt werden. Hierfür können flüssige basische Stoffe zugegeben oder die saure Reaktionsmischung über einen basischen Ionentauscher geleitet werden. Als nicht flüchtige Säuren sind anorganische oder organische Säuren wie z.B. Schwefelsäure, Halogenwasserstoffsäuren, wie z.B. Salzsäure, Perchlorsäure, Trifluoressigsäure, Toluolsulfonsäure, Essigsäure, Propionsäure, Buttersäure, iso-Buttersäure, Valeriansäure, Acrylsäure, oder saure Salze (wie z.B. NaHSO₄) geeignet. Die Säure kann auch als Feststoff in der Kolonne fixiert sein, z.B. als saurer Ionentauscher wie z.B. die Katapack-Kolonneneinbauten der Fa. Sulzer. Bevorzugt werden aber unter den Reaktionsbedingungen flüchtige Säuren und ganz bevorzugt Salpetersäure verwendet.

Gegebenenfalls kann am Kopf der Kolonne ein Hilfsstoff zur Verbesserung der Phasentrennung zwischen wässriger und organischer Phase zugegeben werden. Als solche Hilfsstoffe eignen sich inerte Stoffe, die in Mischung mit Wasser einen niedrigeren Siedepunkt als die Einsatzstoffe aufweisen wie z.B. Kohlenwasserstoffe (Pentan, Hexan, Cyclohexan).

Das Reaktionsvolumen der Reaktionskolonne kann durch den Einbau zusätzlicher Behältnisse vergrößert werden. Auch in den Verdampferstufen kann durch den Einbau von Behältnissen zusätzliches Volumen bereitgestellt werden.

### Beispiel 1

Die Apparatur besteht aus einer Reaktionskolonne K1 mit einem Innendurchmesser von 43 mm, die mit 1,44 m Sulzer-CY-Packung gefüllt ist. Der Druck am Kopf der Kolonne beträgt 89 mbar. Am Kopf der Kolonne befindet sich ein Kondensator W4 und ein Phasentrenngefäß B1. Aus der sich abscheidenden wässrigen Unterphase werden standgeregelt 38,1 g/h abgepumpt, die organische Oberphase läuft frei auf den Kopf der Kolonne K1 zurück. Die Menge dieses Rückflusses wird mit einem Massendurchflussmesser gemessen und beträgt 1490 g/h. Die aus dem Sumpf ablaufende Flüssigkeit gelangt in einen Verdampfer W1. Dort werden 2469 g/h verdampft und die Dämpfe im Kondensator W2 kondensiert. Der Druck im Kondensator W2 beträgt 88 mbar. Zu der im Kondensator W2 gewonnen Flüssigkeit werden 1,7 g/h einer 0,1 molaren wässrigen Salpetersäure, 155,5 g/h frisches 3-Methyl-2-Butenal, 170,8 g/h frisches Prenol (3-Methyl-2-buten-1-ol) und 225,9 g/h eines verunreinigten Prenols zugegeben. Das verunreinigte Prenols enthält 84,4 % Prenol, 2,2 % 3-Methyl-2-Butenal und 13,4 % Verunreinigungen. Die Mischung wird in einen Reaktor C1 gegeben, der aus einem durch 5 Lochböden unterteilten Rohr mit einem Gesamtvolumen von 3,2 1 besteht. Die Temperatur im Reaktor C1 beträgt 30°C. Die aus dem Reaktor C1 austretende Flüssigkeit wird in die Kolonne K1 in einer Packungshöhe von 0,48 m zugegeben. Die aus dem Verdampfer W1 austretende Flüssigkeit wird in den Verdampfer W3 gegeben. Der Druck im Verdampfer W3 ist 95 mbar. Die Temperatur im Verdampfer W3 betrug 110°C. Die aus dem Verdampfer W3 aufsteigenden Dämpfe werden in den Sumpf der Kolonne K1 geleitet. Aus dem Verdampfer W3 laufen 514,1 g/h Flüssigkeit ab, die 71,6 % 3-Methyl-2-butenal-diprenylacetal, 13,6 % Prenol und 1,4 % 3-Methyl-2-Butenal enthalten. Daraus ergibt sich eine Selektivität für Prenol von 91,2 % und für 3-Methyl-2-Butenal von 82,5 %. In den Verdampferstufen W1 und W3 befinden sich jeweils 2,1 l Flüssigkeit.

### Beispiel 2

Die Apparatur besteht aus einer Reaktionskolonne K1 mit einem Innendurchmesser von 43 mm, die mit 1,44 m Sulzer-CY-Packung gefüllt ist. Der Druck am Kopf der Kolonne beträgt 92 mbar. Am Kopf der Kolonne befindet sich ein Kondensator W4 und ein Phasentrenngefäß B1. Aus der sich abscheidenden wässrigen Unterphase werden standgeregelt 59,1 g/h abgepumpt. Aus der organischen Phase werden 8,8 g/h als Ausschleusung entnommen, der Rest der organische Oberphase läuft frei auf den Kopf der Kolonne K1 zurück. Die Menge dieses Rückflusses wird mit einem Massendurchflussmesser gemessen und beträgt 1396 g/h. Die aus dem Sumpf ablaufende Flüssigkeit gelangt in einen Verdampfer W1. Dort werden 808 g/h verdampft und die Dämpfe im Kondensator W2 kondensiert. Der Druck im Kondensator W2 beträgt 90 mbar. Zu der im Kondensator W2 gewonnen Flüssigkeit werden 249,5 g/h frisches 3-Methyl-2-Butenal, 270,5 g/h frisches Prenol (3-Methyl-2-buten-1-ol) und 307,3 g/h eines verunreinigten Prenols zugegeben. Das verunreinigte Prenol enthält 80,8 % Prenol, 1,4 % 3-Methyl-2-Butenal und 17,8 % Verunreinigungen. Die Mischung wird auf den Kopf der Kolonne K1 zugegeben. In den Sumpf der Kolonne K1 wurden 1,1 g/h einer 1 molaren wässrigen Salpetersäure zugegeben. Die aus dem Verdampfer W1 austretende Flüssigkeit wird in den Verdampfer W3 gegeben. Der Druck im Verdampfer W3 ist 95 mbar. Die Temperatur im Verdampfer W3 beträgt 105°C. Die aus dem Verdampfer W3 aufsteigenden Dämpfe werden in den Sumpf der Kolonne K1 geleitet. Aus dem Verdampfer W3 liefen 759,4 g/h Flüssigkeit ab, die 70,8 % 3-Methyl-2-butenaldiprenylacetal, 10,2 % Prenol und 1,4 % 3-Methyl-2-Butenal enthielt. Daraus ergibt sich eine Selektivität für Prenol von 88,0 % und für 3-Methyl-2-Butenal von 78,0 %. In den Verdampferstufen W1 und W3 befinden sich jeweils 0,4 1 Flüssigkeit.

### Beispiel 3

Es wird die im Beispiel 2 beschriebene Apparatur verwendet. Aus dem Phasentrenngefäß werden 47,2 g/h wäßrige Phase und 9,1 g/h organische Phase entnommen. Die Menge des Rückflusses ist 1398 g/h. Im Verdampfer W1 werden 2128 g/h verdampft. Es werden 199,7 g/h frisches 3-Methyl-2-Butenal, 190,0 g/h frisches Prenol und 298 g/h eines verunreinigten Prenols zugegeben und die Mischung auf den Kopf der Kolonne K1 zugegeben. Das verunreinigte Prenol enthielt 79,4 % Prenol, 2,2 % 3-Methyl-2-Butenal und 18,4 % Verunreinigungen. In den Verdampfer W3 wurden 2,5 g/h einer 0,3 gew.-%igen Salpetersäure zugegeben. Die Temperatur im Verdampfer W3 betrug 100,1°C. Aus dem Verdampfer W3 läuft 632,1 g/h Flüssigkeit ab, die 72,9 % 3-Methyl-2-butenal-diprenylacetal, 12,0 % Prenol und 1,6 % 3-Methyl-2-Butenal enthielt. Daraus ergibt sich eine Selektivität für Prenol von 94,7 % und für 3-Methyl-2-Butenal von 82,7 %. In den Verdampferstufen W1 und W3 befinden sich jeweils 1,0 l Flüssigkeit.

## Patentansprüche

1. Verfahren zur Herstellung von ungesättigten Acetalen der allgemeinen Formel I 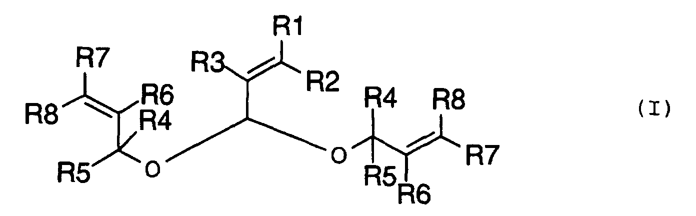 in der
R1 bis R7 unabhängig voneinander Wasserstoff, einen geradkettigen oder verzweigten, gegebenenfalls substituierten C₁-C₆-Alkylrest bedeuten und
R8 Wasserstoff, einen gesättigten oder einen ein oder mehrfach ungesättigten, geradkettigen oder verzweigten, gegebenenfalls substituierten C₁-C₁₂-Alkylrest oder einen gegebenenfalls substituierten 3 bis 12-gliedrigen gesättigten oder einen ein oder mehrfach ungesättigten Carbocyclus bedeutet,
durch Umsetzen von einem Mol eines Aldehyds der Formel II 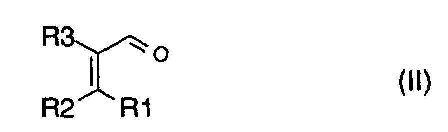 in der R1 bis R3 die oben angegebene Bedeutung haben mit mindestens 1 mol eines Alkohols der Formel III 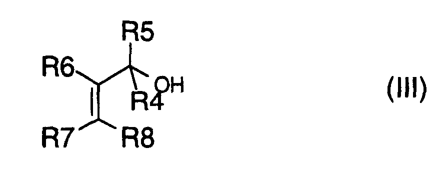 in der R5 bis R8 die oben angegebene Bedeutung haben, in Gegenwart katalytischer Mengen an Säure und unter Abtrennung des bei der Reaktion gebildeten Wassers, **dadurch gekennzeichnet daß** man die Reaktanten in einer Reaktionskolonne nur teilweise umsetzt, das erhaltene Acetal in mindestens zwei aufeinanderfolgenden Verdampferstufen aufkonzentriert und die zurückgewonnenen Reaktanten wieder in die Reaktionskolonne zurückführt.

2. Verfahren nach Anspruch 1, **dadurch gekennzeichnet, daß** der Aldehyd der Formel II, ausgewählt ist aus der Gruppe Acrolein, 2-Buten-1-al, 2-Methyl-2-buten-1-al, 3-Methyl-2-buten-1-al, 2-Methyl-4-methoxy-2-buten-1-al, 2-Methyl-4-methoxy-2-buten-1-al, 3-Isopropyl-2-buten-1-al.

3. Verfahren nach Anspruch 1, **dadurch gekennzeichnet, daß** der Alkohol ausgewählt ist aus der Gruppe 2-Propen-1-ol, 2-Buten-1-ol, 2-Methyl-3-buten-2-ol, 3-Methyl-2-buten-1-ol, Geraniol, 2-Methyl-2-propen-1-ol.

4. Verfahren nach einem der Ansprüche 1 bis 3, **dadurch gekennzeichnet, daß** der in der ersten Verdampferstufe zurückgewonnene Teil der Reaktanten im oberen Teil der Reaktionskolonne zugegeben wird und der in der zweiten Verdampferstufe zurückgewonnenen Teil der Reaktanten auf die unterste Stufe der Reaktionskolonne zugegeben wird.

5. Verfahren nach einem der Ansprüche 1 bis 4, **dadurch gekennzeichnet, daß** das Acetal auf eine Konzentration von 30 Gew.-% bis 99,9 Gew.-% aufkonzentriert wird.

6. Verfahren nach einem der Ansprüche 1 bis 5, **dadurch gekennzeichnet, daß** die Menge der in der ersten Verdampferstufezurückgeführten Reaktanten zwischen dem doppelten und dem dreißigfachen der Menge des frisch zugegebenen Aldehyds beträgt.

7. Verfahren nach einem der Ansprüche 1 bis 6, **dadurch gekennzeichnet, daß** die zugegebene Menge an frisch zugegebenem Alkohol so geregelt wird, daß das Verhältnis aus Alkohol zu Aldehyd zwischen 1 und 3 beträgt.

8. Verfahren nach einem der Ansprüche 1 bis 7, **dadurch gekennzeichnet, daß** im oberen Teil der Destillationskolonne eine nicht flüchtige Säure zugegeben wird.

9. Verfahren nach einem der Ansprüche 1 bis 8, **dadurch gekennzeichnet, daß** die Säure im unteren Teil der Destillationskolonne oder in den Verdampferstufen durch Neutralisation oder Ionenaustausch entfernt wird.

10. Verfahren nach einem der Ansprüche 1 bis 9, **dadurch gekennzeichnet, daß** in der Reaktionskolonne katalytisch aktive Festkörper angeordnet sind.

11. Verfahren nach einem der Ansprüche 1 bis 10, **dadurch gekennzeichnet, daß** die Menge des Rückflusses pro 1000 kg Aldehyd 200 bis 50 000 kg beträgt.

12. Verfahren nach einem der Ansprüche 1 bis 11, **dadurch gekennzeichnet, daß** der Kopfdruck der Kolonne K1 zwischen 2 mbar und 400 mbar liegt, insbesondere so, dass sich am Kopf eine Kondensationstemperatur über 0°C ergibt.

13. Verfahren nach einem der Ansprüche 1 bis 12, **dadurch gekennzeichnet, dass** die Temperatur im Reaktor C1 zwischen -20°C und 100°C liegt.

## Claims

1. A process for the preparation of unsaturated acetals of the formula I 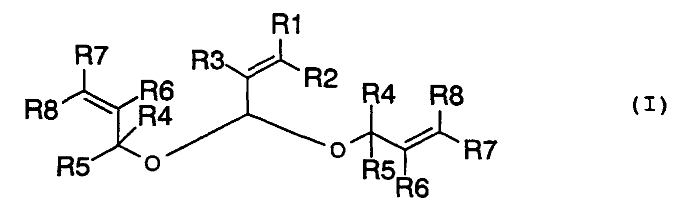 in which
R1 to R7 independently of one another are hydrogen, a straight-chain or branched, optionally substituted C₁-C₆-alkyl radical and
R8 is hydrogen, a saturated or a mono- or polyunsaturated, straight-chain or branched, optionally substituted C₁-C₁₂-alkyl radical or an optionally substituted 3- to 12-membered saturated or a mono- or polyunsaturated carbocycle,
by reacting one mole of an aldehyde of the formula II 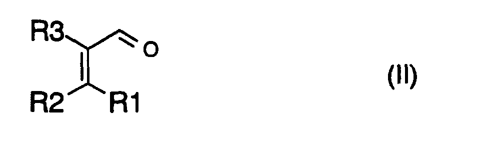 in which R1 to R3 are as defined above, with at least 1 mol of an alcohol of the formula III 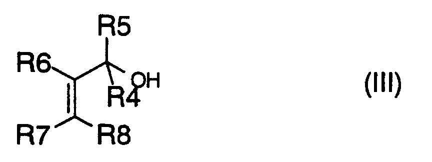 in which R5 to R8 are as defined above, in the presence of catalytic amounts of acid and with removal of the water formed during the reaction, which comprises only partially reacting the reactants in a reaction column, concentrating the resulting acetal in at least two successive evaporation stages, and returning the recovered reactants to the reaction column.

2. A process as claimed in claim 1, wherein the aldehyde of the formula II is chosen from the group acrolein, 2-buten-1-al, 2-methyl-2-buten-1-al, 3-methyl-2-buten-1-al, 2-methyl-4-methoxy-2-buten-1-al, 2-methyl-4-methoxy-2-buten-1-al, 3-isopropyl-2-buten-1-al.

3. A process as claimed in claim 1, wherein the alcohol is chosen from the group 2-propen-1-ol, 2-buten-1-ol, 2-methyl-3-buten-2-ol, 3-methyl-2-buten-1-ol, geraniol, 2-methyl-2-propen-1-ol.

4. A process as claimed in any of claims 1 to 3, wherein the fraction of the reactants recovered in the first evaporation stage is added in the upper part of the reaction column, and the fraction of reactants recovered in the second evaporation stage is added to the lowest stage of the reaction column.

5. A process as claimed in any of claims 1 to 4, wherein the acetal is concentrated to a concentration of from 30% by weight to 99.9% by weight.

6. A process as claimed in any of claims 1 to 5, wherein the amount of the reactants recovered in the first evaporation stage is between 2 and 30 times the amount of freshly added aldehyde.

7. A process as claimed in any of claims 1 to 6, wherein the amount of freshly added alcohol is regulated such that the ratio of alcohol to aldehyde is between 1 and 3.

8. A process as claimed in any of claims 1 to 7, wherein a nonvolatile acid is added in the upper part of the distillation column.

9. A process as claimed in any of claims 1 to 8, wherein the acid in the lower part of the distillation column or in the evaporation stages is removed by neutralization or ion exchange.

10. A process as claimed in any of claims 1 to 9, wherein catalytically active solids are arranged in the reaction column.

11. A process as claimed in any of claims 1 to 10, wherein the amount of reflux is 200 to 50 000 kg per 1000 kg of aldehyde.

12. A process as claimed in any of claims 1 to 11, wherein the head pressure of the column K1 is between 2 mbar and 400 mbar, in particular such that the condensation temperature is above 0°C at the head.

13. A process as claimed in any of claims 1 to 12, wherein the temperature in the reactor C1 is between -20°C and 100°C.

## Revendications

1. Procédé pour la préparation d'acétals insaturés de formule générale I 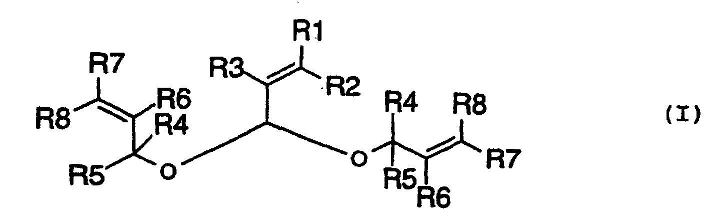 dans laquelle
R1 à R7 désignent, indépendamment l'un de l'autre, l'hydrogène, un reste alkyle en C₁-C₆ linéaire ou ramifié, éventuellement substitué, et
R8 représente l'hydrogène, un reste alkyle en C₁-C₁₂ saturé ou une ou plusieurs fois insaturé, linéaire ou ramifié, éventuellement substitué, ou un carbocycle ayant 3 à 12 chaînons, saturé ou une ou plusieurs fois insaturé, éventuellement substitué,
par réaction d'une mol d'un aldéhyde de formule II 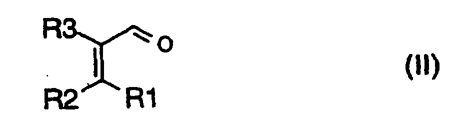 dans laquelle R1 à R3 ont la signification susdite avec au moins une mole d'un alcool de formule III 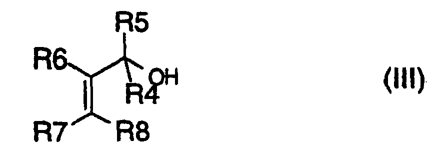 dans laquelle R5 à R8 ont la signification indiquée plus haut, en présence de quantités catalytiques d'acide et avec séparation de l'eau formée dans la réaction, **caractérisé par le fait qu'**on fait réagir seulement partiellement les produits réagissants dans une colonne de réaction, on concentre l'acétal formé dans au moins deux étages d'évaporation disposés l'un au-dessus de l'autre et on recycle les produits réagissants récupérés dans la colonne de réaction.

2. Procédé selon la revendication 1, **caractérisé par le fait que** l'aldéhyde de formule II est choisi dans le groupe consistant en acroléine, 2-butène-1-al, 2-méthyl-2-butène-1-al, 3-méthyl-2-butène-1-al, 2-méthyl-4-méthoxy-2-butène-1-al, 2-méthyl-4-méthoxy-2-butène-1-al, 3-iso-propyl-2-butène-1-al.

3. Procédé selon la revendication 1, **caractérisé par le fait que** l'alcool est choisi dans le groupe consistant en 2-propène-1-ol, 2-butène-1-ol, 2-méthyl-3-butène-2-ol, 3-méthyl-2-butène-1-ol, géraniol, 2-méthyl-2-propène-1-ol.

4. Procédé selon l'une des revendications 1 à 3, **caractérisé par le fait que** la partie des produits réagissants récupérée dans le premier étage d'évaporation est introduite dans la partie supérieure de la colonne de réaction et la partie des produits réagissants récupérée dans le deuxième étage d'évaporation est introduite sur l'étage inférieur de la colonne de réaction.

5. Procédé selon l'une des revendications 1 à 4, **caractérisé par le fait que** l'acétal est concentré à une concentration de 30% en poids à 99,9% en poids.

6. Procédé selon l'une des revendications 1 à 5, **caractérisé par le fait que** la quantité des produits réagissants réintroduits dans le premier étage d'évaporation vaut entre le double et le triple de la quantité de l'aldéhyde fraîchement ajouté.

7. Procédé selon l'une des revendications 1 à 6, **caractérisé par le fait que** la quantité ajoutée d'alcool fraîchement ajouté est réglée de telle manière que le rapport de l'alcool à l'aldéhyde se situe entre 1 et 3.

8. Procédé selon l'une des revendications 1 à 7, **caractérisé par le fait qu'**on introduit un acide non volatil dans la partie supérieure de la colonne de distillation.

9. Procédé selon l'une des revendications 1 à 8, **caractérisé par le fait que** l'acide est éliminé dans la partie inférieure de la colonne de distillation ou dans l'étage d'évaporation par neutralisation ou échange d'ions.

10. Procédé selon l'une des revendications 1 à 9, **caractérisé par le fait que** des solides catalytiquement actifs sont disposés dans la colonne de réaction.

11. Procédé selon l'une des revendications 1 à 10, **caractérisé par le fait que** la quantité de reflux vaut 200 à 50 000 kg pour 1000 kg d'aldéhyde.

12. Procédé selon l'une des revendications 1 à 11, **caractérisé par le fait que** la pression en tête de la colonne K1 vaut entre 2 mbar et 400 mbar, en particulier de telle manière que l'on ait en tête une température de condensation supérieure à 0°C.

13. Procédé selon l'une des revendications 1 à 12, **caractérisé par le fait que** la température dans le réacteur C1 se situe entre -20°C et 100°C.
